# FASCICULE DE BREVET EUROPEEN

(11) **EP 2 184 048 B1**
(45) Date de publication et mention de la délivrance du brevet: **25.02.2015**
(21) Numéro de dépôt: 09174776.6
(22) Date de dépôt: 02.11.2009
(51) Int. Cl.: A61K 8/06, A61K 8/81, A61K 8/895, A61Q 19/00

(54) **Emulsion huile-dans-eau contenant un polymère amphiphile et un elastomère siliconé**
Öl-in-Wasser-Emulsion, die ein amphiphiles Polymer und ein Silikonelastomer enthält
Oil-in-water emulsion containing an amphiphilic polymer and a siliconised elastomer

(30) Priorité: 07.11.2008 FR 0857570
(43) Date de publication de la demande: 12.05.2010
(73) Titulaire: L'Oréal, 75008 Paris (FR)
(72) Inventeur: Moneuze, Gaelle, 75020, Paris (FR); Cotton, Thierry, 75013, Paris (FR); Lavaud, Brigitte, 75016, Paris (FR)
(74) Mandataire: Duvert, Sandra

(56) Documents cités:
- EP-A- 1 055 406
- EP-A- 1 415 645
- WO-A-2007/141142

## Description

La présente demande se rapporte à une composition sous forme d'émulsion huile-dans-eau, contenant au moins un polymère amphiphile particulier.
Pour diverses raisons liées en particulier à un meilleur confort d'utilisation (douceur, émollience et autres), les compositions cosmétiques actuelles se présentent le plus souvent sous la forme d'une émulsion du type huile-dans-eau (H/E) constituée d'une phase continue dispersante aqueuse et d'une phase discontinue dispersée huileuse ou d'une émulsion du type eau-dans-huile (E/H) constituée d'une phase continue dispersante huileuse et d'une phase discontinue dispersée aqueuse. Les émulsions H/E sont les plus demandées dans le domaine cosmétique du fait qu'elles comportent comme phase externe, une phase aqueuse, ce qui leur confère, lors de l'application sur la peau, un toucher plus frais, moins gras et plus léger que les émulsions E/H.
En outre pour améliorer les propriétés de toucher de ces émulsions, on peut utiliser des élastomères siliconés ; ces composés apportent de la douceur mais peuvent être difficiles à stabiliser dans l'émulsion et nécessitent l'utilisation de tensioactifs .
Toutefois l'addition de tensioactifs entraîne une perte de transparence du produit car l'émulsion présente généralement une taille des gouttes d'huile ou globules huileux de l'ordre du micron. Ces globules huileux diffusent donc fortement la lumière et l'émulsion présente alors une couleur blanche
Il est connu par exemple du document EP 1415645 des émulsions comprenant un élastomère siliconé et des polymères amphiphiles dérivés d'AMPS réticulés comme les produits commercialisés sous la dénomination Hostacerin AMPS vendu par la société Hoechst. Ces polymères réticulés conduisent à des émulsions présentant un aspect plus translucide mais qui sont difficiles à stabiliser dans le cas où l'on veut obtenir des textures fluides, car un phénomène de crémage de l'émulsion se produit.

Il existe donc un besoin de réaliser des émulsions huile dans eau qui présentent un aspect translucide satisfaisant, confèrent à la peau un toucher doux, frais et léger, donnent un effet non gras sur la peau et qui sont stables quelle que soit leur viscosité et peuvent donc être formulées dans une large gamme de texture (fluide sprayable à crème consistante).

La demanderesse a trouvé de façon surprenante la possibilité de réaliser des émulsions huile dans eau comprenant au moins un polymère amphiphile dérivé d'AMPS non réticulé et un organopolysiloxane élastomère qui répondent à ce besoin. Les émulsions obtenues sont translucides et peuvent se présenter aussi bien sous la forme de fluides que de crèmes consistantes . Elles se présentent en particulier sous la forme d'un gel émulsionné.

Ainsi, la présente invention concerne une composition pour application topique, sous forme d'émulsion huile-dans-eau comprenant une phase huileuse dispersée dans une phase aqueuse, **caractérisée en ce qu**'elle comprend :
- au moins un polymère amphiphile non réticulé comprenant :
   (a) de 80 à 99% en moles de motif acide 2-acrylamido 2- 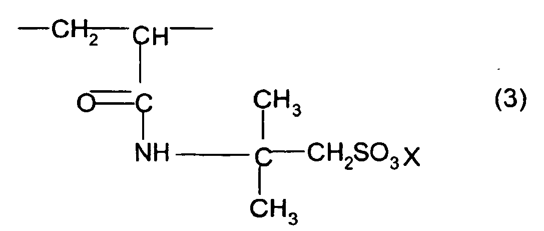 méthylpropane sulfonique (AMPS) de formule (3) suivante :
      dans laquelle X est un proton, un cation de métal alcalin, un cation alcalino-terreux ou l'ion ammonium, ; étant entendu que lorsque X représente un cation métallique alcalino terreux, il partage 2 charges positives avec 2 groupements SO3 ;
   (b) et de 1 à 20% en moles, et de préférence de 1 à 15% en moles de motif de 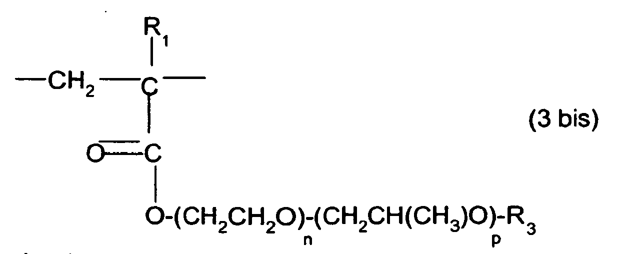 formule (3 bis) suivante :
   dans laquelle n et p, indépendamment l'un de l'autre désignent un nombre de moles et varie de 0 à 30, sous réserve que n + p soit inférieur ou égal à 30; R₁ désigne un atome d'hydrogène, un radical alkyle linéaire ou ramifié en C₁-C₆ et R₃ désigne un alkyle linéaire ou ramifié comportant m atomes de carbone allant de 6 à 30, et
- au moins un organopolysiloxane élastomère.

On entend ici par « application topique », une application externe sur les matières kératiniques, que sont notamment la peau, le cuir chevelu, les cils, les sourcils, les ongles, les muqueuses et les cheveux.

La composition selon l'invention étant destinée à une application topique, elle contient un milieu physiologiquement acceptable, c'est-à-dire compatible avec les matières kératiniques telles que la peau, les muqueuses, les fibres kératiniques comme les cils, les cheveux et le cuir chevelu.

La composition selon l'invention présente l'avantage d'avoir une bonne innocuité et de bonnes propriétés cosmétiques, c'est-à-dire une texture homogène et agréable à l'application. En outre, elle est bien stable dans le temps. Une émulsion est stable si aucune évolution de son aspect macroscopique, microcoscopique et de ses caractéristiques physico-chimiques (taille des gouttes, pH, viscosité) n'est observée après une mise en conservation à température ambiante pendant une durée de 15 jours.

La composition selon l'invention présente avantageusement une taille moyenne des globule huileux allant de 15 à 500 µm.
La taille moyenne des globule huileux peut être mesurée au microscopique de type LEICA DM LB2, avec un objectif permettant un grossissement X10, selon le protocole suivant : on dépose sur une lame de verre une pointe du produit à mesurer à l'aide d'une spatule fine, puis on écrase une lamelle sur l'échantillon. L'échantillon ainsi préparé est observé à l'oeil via l'objectif du microscope.
On repère en se déplaçant sur l'échantillon les gouttes dont la taille est la plus représentative de la population observée.
A l'aide du logiciel de traitement de l'image du microscope, on mesure ensuite avec une réglette le diamètre de la goutte repérée, directement sur l'écran de l'ordinateur relié au microscope. Ce diamètre est le diamètre moyen ou taille moyenne des globules huileux de l'émulsion.
On définit de la même manière le diamètre minimum, et le diamètre maximum de la population des globules de l'échantillon considéré.

La taille moyenne des globules huileux peut aller de 15 à 500 µm, préférentiellement de 15 à 300 µm et encore mieux de 15 à 250 µm.

Les émulsions selon l'invention sont translucides, en particulier, elles présentent une transmittance à la lumière à une longueur d'onde égale à 500 nm, à travers un échantillon de 50µm d'épaisseur, d'au moins 1,5 fois plus élevée qu'une émulsion de même composition dont le diamètre des gouttes est inférieure à 15 µm.
La transmittance est mesurée à l'aide d'un spectrophotomètre UV Visible Carry 600, à une longueur d'onde égale à 500 nm. L'émulsion est placée entre deux lames de quartz d'épaisseur 0,05 mm dont l'une comprend une encoche de 50 microns de profondeur.

La viscosité des dispersions obtenues peut aller de très fluide à très visqueuse (crème) et elle est ajustée notamment en fonction de la teneur en polymère introduite et du taux de phase huileuse émulsionnée. La composition de l'invention présente une viscosité pouvant aller par exemple de 0,01 Pa.s à 100 Pa.s à une température de 25°C, la viscosité étant mesurée à l'aide d'un Rhéomat 180 (Société LAMY), équipé d'un mobile MS-R1, MS-R2, MS-R3, MS-R4 ou MS-R5 choisi en fonction de la consistance de la composition, tournant à un vitesse de rotation de 200 t.min-1.

L'invention a également pour objet une procédé de traitement cosmétique des matières kératiniques, **caractérisé en ce qu**'on applique sur les matières kératiniques, une composition cosmétique telle que décrite ci-dessus.

Selon un autre aspect, l'invention a pour objet l'utilisation de l'association
- d'au moins un polymère amphiphile non réticulé comprenant :
   (a) de 80 à 99% en moles de motif acide 2-acrylamido 2-méthylpropane sulfonique (AMPS) de formule (3) suivante : 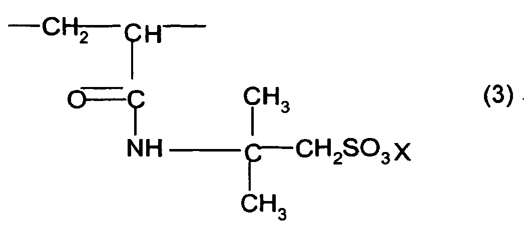 dans laquelle X⁺ est un proton, un cation de métal alcalin, un cation alcalino-terreux ou l'ion ammonium, ; étant entendu que lorsque X représente un cation métallique alcalino terreux, il partage 2 charges positives avec 2 groupements SO3 ;
   (b) et de 1 à 20% en moles, et de préférence de 1 à 15% en moles de motif de formule (3 bis) suivante : 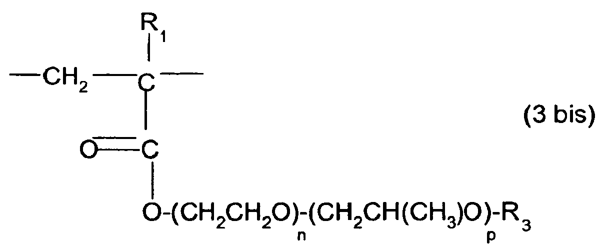
   dans laquelle n et p, indépendamment l'un de l'autre désignent un nombre de moles et varie de 0 à 30, sous réserve que n + p soit inférieur ou égal à 30; R₁ désigne un atome d'hydrogène, un radical alkyle linéaire ou ramifié en C₁-C₆ et R₃ désigne un alkyle linéaire ou ramifié comportant m atomes de carbone allant de 6 à 30, et
- d'au moins un organopolysiloxane élastomère,
pour obtenir une émulsion huile-dans-eau translucide.

### Polymère amphiphile

Le polymère amphiphile utilisé dans la composition selon l'invention est polymère dérivé d'acide 2-acrylamido-2-méthylpropane-sulfonique (AMPS) comprenant
(a) de 80 à 99% en moles de motif acide 2-acrylamido 2-méthylpropane sulfonique (AMPS) de formule (3) suivante : 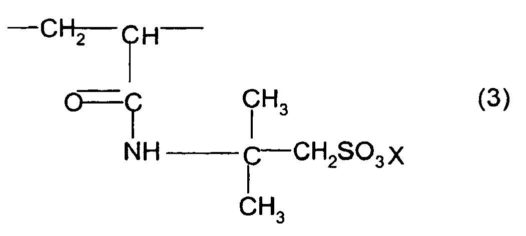 dans laquelle X⁺ est un proton, un cation de métal alcalin, un cation alcalino-terreux ou l'ion ammonium, ; étant entendu que lorsque X représente un cation métallique alcalino terreux, il partage 2 charges positives avec 2 groupements S03 ;
(b) et de 1 à 20% en moles, et de préférence de 1 à 15% en moles de motif de formule (3 bis) suivante : 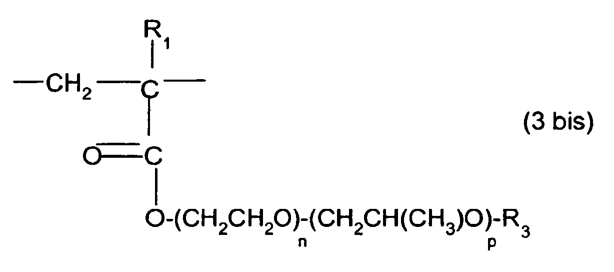
dans laquelle n et p, indépendamment l'un de l'autre désignent un nombre de moles et varie de 0 à 30, de préférence de 1 à 20 sous réserve que n + p soit inférieur ou égal à 30, de préférence inférieur à 25 et encore mieux inférieur à 20 ; R₁ désigne un atome d'hydrogène, un radical alkyle linéaire ou ramifié en C₁-C₆ (de préférence méthyle) et R₃ désigne un groupe alkyle linéaire ou ramifié comportant m atomes de carbone allant de 6 à 30, de préférence de 10 à 25 atomes de carbone.

Par « polymère amphiphile », on entend un polymère qui comprend au moins une partie (ou séquence) hydrophile et au moins une partie (ou séquence) hydrophobe. Ce polymère est hydrosoluble ou hydrodispersible.

Les polymères amphiphiles utilisés dans la composition de l'invention sont hydrosolubles ou hydrodispersibles. On entend par "polymère hydrosoluble ou hydrodispersible", un polymère qui, introduit dans de l'eau à une concentration égale à 1 % en poids, conduit à une solution macroscopiquement homogène dont la transmittance de la lumière, à une longueur d'onde égale à 500 nm, à travers un échantillon de 1 cm d'épaisseur, est d'au moins 10 %, ce qui correspond à une absorbance [abs = -log (transmittance)] inférieure à 1,5.

Les polymères amphiphiles conformes à l'invention ont en général une masse molaire en poids allant de 50 000 à 10 000 000, plus préférentiellement de 100 000 à 8 000 000 et encore plus préférentiellement de 200 000 à 3 000 000.

Les polymères conformes à l'invention sont de façon préférentielle neutralisés partiellement ou totalement par une base minérale telle que, par exemple, soude, potasse, ammoniaque, ou par une base organique telle que la mono-, di- et triéthanolamine, l'aminométhylpropanediol, la N- méthyl-glucamine, les acides aminés basiques comme l'arginine et la lysine, et leurs mélanges.

Les polymères utilisés selon l'invention peuvent être obtenus selon les procédés classiques de polymérisation radicalaire en présence d'un ou plusieurs initiateurs tels que par exemple l'azobisisobutyronitrile (AIBN), l'azobisdiméthylvaléronitrile, le chlorhydrate de 2,2-azobis-[2-amidinopropane] (ABAH = 2,2-azoBis-[2-Amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂0₂ éventuellement en présence de réducteurs.

Les polymères sont notamment obtenus par polymérisation radicalaire en milieu tert-butanol dans lequel ils précipitent. En utilisant la polymérisation dans le tert-butanol, il est possible d'obtenir une distribution de la taille des particules de polymère particulièrement favorable pour ses utilisations.

La réaction de polymérisation peut être conduite à une température comprise entre 0°C et 150°C, de préférence entre 20°C et 100°C, soit à pression atmosphérique, soit sous pression réduite. Elle peut aussi être réalisée sous atmosphère inerte, et de préférence sous azote.

Les polymères amphiphiles d'AMPS utilisés dans la composition selon l'invention sont non-réticulés.

Comme polymères dérivés d'AMPS utilisables dans la composition selon l'invention, on peut citer les polymères préparés à partir de l'acide 2-acrylamido-2-méthylpropane-sulfonique (AMPS) ou l'un de ses sels de sodium ou d'ammonium, avec un ester de l'acide (méth)acrylique et d'un alcool en C10 à C20 oxyéthyléné comprenant de 6 à 25 groupes oxyéthylénés.
On peut citer en particulier les polymères préparés à partir de l'acide 2-acrylamido-2-méthylpropane-sulfonique (AMPS) ou l'un de ses sels de sodium ou d'ammonium, avec un ester de l'acide (méth)acrylique et :
- d'un alcool en C₁₀-C₁₈ oxyéthyléné par 8 moles d'oxyde d'éthylène (Genapol C-080 de la société Clariant),
- d'un alcool oxo en C₁₁ oxyéthyléné par 8 moles d'oxyde d'éthylène (Genapol UD-080 de la société Clariant),
- d'un alcool oxo en C₁₁ oxyéthyléné par 7 moles d'oxyde d'éthylène (Genapol UD-070 de la société Clariant),
- d'un alcool en C₁₂-C₁₄ oxyéthyléné par 7 moles d'oxyde d'éthylène (Genapol LA-070 de la société Clariant),
- d'un alcool en C₁₂-C₁₄ oxyéthyléné par 9 moles d'oxyde d'éthylène (Genapol LA-090 de la société Clariant),
- d'un alcool en C₁₂-C₁₄ oxyéthyléné par 11 moles d'oxyde d'éthylène (Genapol LA-110 de la société Clariant),
- d'un alcool en C₁₆-C₁₈ oxyéthyléné par 8 moles d'oxyde d'éthylène (Genapol T-080 de la société Clariant),
- d'un alcool en C₁₆-C₁₈ oxyéthyléné par 11 moles d'oxyde d'éthylène (Genapol T-110 de la société Clariant),
- d'un alcool en C₁₆-C₁₈ oxyéthyléné par 15 moles d'oxyde d'éthylène (Genapol T-150 de la société Clariant),
- d'un alcool en C₁₆-C₁₈ oxyéthyléné par 20 moles d'oxyde d'éthylène (Genapol T-200 de la société Clariant),
- d'un alcool en C₁₆-C₁₈ oxyéthyléné par 25 moles d'oxyde d'éthylène (Genapol T-250 de la société Clariant),
- d'un alcool en C₁₈-C₂₂ oxyéthyléné par 25 moles d'oxyde d'éthylène,
- d'un alcool en iso-C₁₆-C₁₈ oxyéthyléné par 25 moles d'oxyde d'éthylène.

Selon un mode préféré de réalisation, le polymère amphiphile est un copolymère d'AMPS et de méthacrylate d'alcool en C₁₆-C₁₈ comportant de 6 à 25 groupes oxyéthylénés, obtenus à partir d'acide méthacrylique ou d'un sel d'acide méthacrylique et d'un alcool en C₁₆-C₁₈oxyéthyléné par 6 à 25 moles d'oxyde d'éthylène. Le polymère amphiphile peut également être un copolymère d'AMPS et de méthacrylate d'alcool en C₁₂-C₁₄ comportant de 6 à 25 groupes oxyéthylénés, obtenus à partir d'acide méthacrylique ou d'un sel d'acide méthacrylique et d'un alcool en C₁₂-C₁₄ oxyéthyléné par 6 à 25 moles d'oxyde d'éthylène.

Comme polymères amphiphiles préférés selon la présente invention, on peut citer :
- le copolymère non réticulé obtenu à partir de 92,65% en moles d'AMPS et de 7,35% en moles de méthacrylate d'alcool en C₁₆-C₁₈ comportant 8 groupes oxyéthylénés (Genapol T-080), tel que celui commercialisé par la société Clariant sous la dénomination Aristoflex SNC
- le copolymère non réticulé obtenu à partir de 91,5% en moles d'AMPS et de 8,5% en moles de méthacrylate d'alcool en C₁₂-C₁₄ comportant 7 groupes oxyéthylénés (Genapol LA-070), tel que celui commercialisé par la société Clariant sous la dénomination Aristoflex LNC
- et leurs mélanges.

Ces copolymères sont appropriés pour donner des émulsions stables et se présentant sous des textures très variées, allant du fluide sprayable à la crème avec de très bonnes qualités cosmétiques.

Ces polymères présentent l'avantage d'être peu sensibles aux variations de pH pour des valeurs comprises entre 4 et 8, qui sont les valeurs habituelles des compositions cosmétiques.
La quantité (en matière active) de polymère amphiphile non réticulé tel que décrit plus haut dans la composition selon l'invention peut aller en particulier de 0,01 à 10% en poids par rapport au poids total de la composition, de préférence de 0,05 à 5% en poids et mieux de 0,1 à 3% en poids.

### 2/ Organopolysiloxane élastomère

La composition de l'invention contient au moins un organopolysiloxane élastomère, aussi appelé « élastomère siliconé » dans la suite de la description, de préférence au moins partiellement réticulé. On entend par « élastomère » un matériau solide souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomères utilisés dans la composition selon l'invention sont de préférence partiellement ou totalement réticulés. Ils se présentent sous forme de particules. En particulier, les particules d'organopolysiloxane élastomère ont une taille allant de 0,1 à 500 µm, de préférence de 3 à 200 µm et mieux de 3 à 50 µm. Ces particules peuvent avoir toute forme et par exemple être sphériques, plates ou amorphes.

Quand ils sont inclus dans une phase huileuse, ces organopolysiloxanes élastomères se transforment, selon le taux de phase huileuse utilisé, en un produit d'aspect spongieux lorsqu'ils sont utilisés en présence de faibles teneurs en phase huileuse, ou en un gel homogène en présence de quantités de phase huileuse plus élevées. La gélification de la phase huileuse par ces élastomères peut être totale ou partielle.

Ainsi les élastomères de l'invention peuvent être véhiculés sous forme de gel anhydre constitué d'un organopolysiloxane élastomère et d'une phase huileuse. La phase huileuse utilisée lors de la fabrication du gel anhydre d'organopolysiloxane élastomère contient une ou plusieurs huiles liquides à températures ambiante (25°C) choisies parmi les huiles hydrocarbonées et/ou les huiles de silicone. Avantageusement, la phase huileuse est une phase liquide siliconée, contenant une ou plusieurs huiles choisies parmi les polydiméthylsiloxanes à chaîne linéaire ou cyclique, liquides à température ambiante comportant éventuellement une chaîne alkyle ou aryle pendante ou en bout de chaîne, la chaîne alkyle ayant de 1 à 6 atomes de carbone.

Les organopolysiloxanes élastomères utilisés selon l'invention peuvent être choisis parmi les polymères réticulés décrits dans la demande EP-A-0295886 et parmi ceux décrits dans le brevet US-A-5,266,321.
Ils sont de préférence non émulsionnant. Le terme organopolysiloxanes élastomères "non émulsionnant" définit des élastomères organopolysiloxane ne contenant pas de chaîne hydrophile telle que motifs polyoxyalkylènes ou polyglycérolés.

L'élastomère de silicone est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A) de diorganopolysiloxane contenant au moins deux atomes d'hydrogènes liés chacun à un atome de silicium différent, et (B) de diorganopolysiloxane ayant au moins deux groupements à insaturation éthylénique liés au silicium, notamment en présence (C) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane. L'organopolysiloxane (A) peut avoir une structure chaîne ramifiée, chaîne linéaire, cyclique ou réseau mais la structure chaîne linéaire est préférée. Le composé (A) peut avoir une viscosité allant de l'état liquide à l'état de gomme. De préférence, le composé (A) a une viscosité d'au moins 100 centistokes à 25 °C.
Les organopolysiloxanes (A) peuvent être choisi parmi les méthylvinylsiloxanes, les copolymères méthylvinylsiloxane-diméthylsiloxanes, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B) est en particulier un organopolysiloxane ayant au moins 2 hydrogènes liés au silicium dans chaque molécule et est donc le réticulant du composé (A).
Avantageusement, la somme du nombre de groupements éthyléniques par molécule du composé (A) et le nombre d'atomes d'hydrogène liés au silicum par molécule du composé (B) est d'au moins 4.
Le composé (B) peut être sous toute structure moléculaire, notamment de structure chaîne linéaire, chaîne ramifiée, structure cyclique.
Le composé (B) peut avoir une viscosité à 25 °C allant de 1 à 50 000 centistokes, notamment pour être bien miscible avec le composé (A).
Il est avantageux que le composé (B) soit ajouté en une quantité telle que le rapport moléculaire entre la quantité totale d'atomes d'hydrogène liés au silicium dans le composé (B) et la quantité totale de tous les goupements à insaturation éthylénique dans le composé (A) aille dans la gamme de 1/1 à 20/1.
Le composé (B) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

D'autres groupes organiques peuvent être liés au silicium dans les organopolysiloxane (A) et (B) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un groupe ester carboxylate, un groupe mercapto.

L'élastomère de silicone non émulsionnant est généralement mélangé avec au moins une huile hydrocarbonée et/ou une huile siliconée pour former un gel. Dans ces gels, l'élastomère non émulsionnant est sous forme de particules non-sphériques.
Les organopolysiloxanes élastomères utilisés dans la composition de l'invention peuvent être par exemple ceux commercialisés sous les noms KSG 6 par la société Shin-Etsu ; Trefil E-505C ou Trefil E-506C par la société Dow-Corning ; Gransil (SR-CYC, SR DMF10, SR-DC556) par la société Grant Industries, ou ceux commercialisés sous forme de gels déjà constitués : KSG 15, KSG 16, KSG 17, KSG 18, KSG 26A, KSG 26B, KSG-31", "KSG-32", "KSG-33", "KSG-41", "KSG-42", "KSG-43", "KSG-44" de la société Shin-Etsu ; Gransil SR 5CYC gel, Gransil SR DMF 10 gel, Gransil SR DC556 gel de la société, Gransil RPS de Grant Industries ; 1229-02-167 et 1229-02-168 de la société General Electric.
Selon un mode de réalisation préféré, on utilise des élastomères siliconés portant la dénomination INCI dimethicone/vinyldimethicone copolymer (ou polysilicone-11) en mélange avec une huile siliconée cyclique. On peut citer par exemple le mélange d'organopolysiloxane réticulé/cyclopentasiloxane ou un mélange d'organopolysiloxane réticulé/cyclohexasiloxane comme par exemple le Gransil RPS D5 ou le Gransil RPS D6 de la société Grant Industries

On peut également citer les élastomères commercialisés sous les références DC 9040", "DC9041", "DC 9509", "DC9505", "DC 9506" par la société Dow Corning. On peut aussi utiliser un mélange d'élastomères de silicone, et notamment un mélange de ces produits commerciaux.

Le ou les organopolysiloxanes élastomères utilisés selon l'invention sont présents en une quantité en matière active qui varie selon le but recherché. Cette quantité peut aller par exemple de 0,01 à 10 %, de préférence de 0,5 à 7 % et mieux de 1 à 4 % du poids total de la composition.

### Emulsionnants

Afin de faciliter l'émulsification de la phase huileuse, la composition selon l'invention peut comprendre un ou plusieurs émulsionnants (distincts du polymère amphiphile non réticulé), aussi appelés «co-émulsionnants ».
La quantité (en matière active) d'émulsionnant(s) peut aller par exemple de 0,001% et 5% en poids, de préférence de 0,005% et 2% en poids, et mieux de 0,01% à 1% en poids par rapport au poids total de la composition.
L'émulsionnant est de préférence utilisé à un taux inférieur à 20% en poids par rapport au poids de polymère amphiphile.
L'émulsionnant est de préférence utilisé à un taux inférieur ou égal à 1% en poids par rapport au poids de la composition, de préférence inférieur ou égal à 0,5% en poids et mieux inférieur ou égal à 0,1% en poids. Selon un mode de réalisation, la composition selon l'invention est exempte d'émulsionnant ou co-émulsionnant.

L'émulsionnant peut être choisi parmi les alkylpolyglucosides, les alkyl ester ou ether de polyoxyethylène (POE), les alkyl ester ou ether de glycerol, les alkyl ester ou ether de sorbitan oxyethyléné ou non, les dimethicone copolyols, les gemini, les acylglutamate mono ou disodique.

On peut citer en particulier :
- les esters de glycérol tels que les mono ou polyalkylesters ou éthers de glycérol tels que décrits dans les documents EP1010416 et EP1010414, le mono-isostéarate de glycéryle, tel que le produit commercialisé sous la dénomination de Peceol Isostarique par la société Gattefosse, , l'isostéarate polyglycérolé (4 moles) vendu sous la dénomination Isolan G134 par la société Goldschmidt, le diisostéarate polyglycérolé (3 moles) vendu sous la dénomination Lameform TGI par la société Cognis et le distéarate polyglycérolé (2 moles) vendu sous la dénomination Emalex PGSA par la société Nihon emulsion.
- les esters et éthers de polyéthylène glycol , tels que les alkylesters et éthers de polyéthylène glycol tels que décrits dans les documents EP1120101 et EP1016453, l'oleth 50 vendu sous la dénomination de Emalex 550 par la société Nihhon Emulsion, l'oleth 20 vendu sous la dénomination de Brij 98 par la société Uniqema, les ceteth 2 et 10 vendu sous la dénomination de Brij 52 et 56 par la société Uniqema, le laureth 23 vendu sous la dénomination de Brij 35 par la société Uniqema et le PEG 8 stearate vendu sous la dénomination de Myrj 45 par la société Uniqema, l'isostéarate de PEG-8 tel que le produit commercialisé sous la dénomination Prisorine 3644 par la société Uniquema, le PEG 20 stéarate et le PEG 40 stéarate vendus sous la dénomination de Myrj 49 et de Myrj 52 par la société Uniqema.
On peut encore citer le composés suivants vendus par la société Uniqema :

| Nom commercial | Nom INCI |
|---|---|
| Brij 35 | Laureth 23 |
| Brij 30 | Laureth 4 |
| Brij 96 | Oleth 10 |
| Brij 56 | Ceteth 10 |
| Brij 98 | Oleth 20 |
| Brij 76 | Steareth 10 |
| Brij 72 | Steareth 2 |
| Brij 52 | Ceteth 2 |
| Brij 78 | Steareth 20. |

- Les esters ou éthers de sorbitane tels que les mono ou polyalkylesters ou éthers de sorbitan oxyéthylénés ou non tels que décrits dans le document EP1010415, ou encore les produits suivants vendus par la société Uniqema :
Exemples : Nom commercial Nom INCI

| | |
|---|---|
| Tween 21 | Polysorbate 21 |
| Tween 40 | Polysorbate 40 |
| Tween 80 | Polysorbate 80 |
| Tween 60V | Polysorbate 60 |
| Tween 61V | Polysorbate 61. |

Citons également l'isostéarate de sorbitane tel que le produit commercialisé sous la dénomination Arlacel 987 par la société Uniqema, l'isostérate de sorbitan et de glycérol, tel que le produit commercialisé sous la dénomination Arlacel 986 par la société Uniqema, le sesquioleate de sorbitane tel que le produit vendu sous la dénomination Arlacel 83V par la société Uniqema, le laurate de sorbitane, le monopalmitate de sorbitane, l'oléate de sorbitane, le trioléate de sorbitane, le monostéarate de sorbitane et le tristéarate de sorbitane tels que les produits commercialisés sous la dénomination Span 20, Span 40, Span 80V, Span 85V, Span 60 et Span 65V par la société Uniqema.
- Les mono ou polyalkylesters ou éthers de sucre tels que les mono ou polyalkylesters ou éthers de sucres tels que décrits dans le brevet US6689371. On peut citer par exemple l'isostéarate de méthylglucose tel que l'Isolan-IS de la société Degussa Goldschmidt ou encore le sucrose distéarate tel que le Crodesta F50 vendu par la société Croda, et le sucrose stearate tel que le Ryoto sugar ester S 1570 vendu par la société Mitsubishi Kagaku Foods.
- Les alkenyl succinates alkoxylés par exemple tels que décrits dans le document EP1025898.
- Les alcools gras tels que les alcools gras ayant de 8 à 26 atomes de carbone comme l'alcool cétylique, l'alcool stéarylique et leur mélange (alcool cétéarylique), l'octyl dodécanol, le 2-butyloctanol, le 2-hexyldécanol, le 2-undécylpentadécanol, l'alcool oléique et leurs mélanges.
- Les dérivés siliconés comme les diméthicone copolyols tels que le mélange de cyclomethicone et de dimethicone copolyol, vendu sous la dénomination « DC 5225 C » par la société Dow Corning, et les alkyl dimethicone copolyols tels que le laurylmethicone copolyol vendu sous la dénomination « Dow Corning 5200 Formulation Aid » par la société Dow Corning et le cetyldimethicone copolyol vendu sous la dénomination « Abil EM 90 » par la société Goldschmidt, ou le mélange polyglycéryl-4 isostéarate/cétyl dimethicone copolyol/hexyllaurate vendu sous la dénomination « Abil WE 90 » par la société Goldschmidt.
- Les alkenyl succinates alkoxylés tels que par exemple ceux qui sont décrits dans le document EP1025898.
- les alkyl esters phosphoriques_tels que par exemple ceux qui sont décrits dans le document EP1013338.
- Les alkyl éther citrates tels que par exemple ceux qui sont décrits dans le document EP1020219.
- Les lipoaminoacides et leurs sels tels que les acylglutamates mono- et di- sodiques comme par exemple le mono sodium stéaroyl glutamate (Amisoft HS-11 PF) et le disodium stearoyl glutamate (Amisoft HS-21 P) vendus par la société Ajinomoto.
- les alkyls phosphates et leurs sels tels que les sels alcalins du dicétyl- et du dimyristylphosphate, ou encore le potassium cétyl phosphate tel que l'Amphisol K vendu par la société DSM Nutritional Products.
- Les dérivés du cholestérol tels que les sels alcalins du cholestérol sulfate, les sels alcalins du cholestérol phosphate.
- Les sels d'ammonium de l'acide phosphatidique.
- Les phospholipides.
- Les dérivés alklsulfoniques tels que décrits dans le brevet document EP1120101.

Selon un mode préféré de l'invention, le co-émulsionnant est choisi parmi les esters de glycéryle (isostéarate de glycéryle), les esters de sorbitane (Polysorbate 60) et les esters de polyéthylène glycol (PEG 8 isostéarate).

### Phase aqueuse

La phase aqueuse de la composition selon l'invention comprend de l'eau et éventuellement un ou plusieurs composés miscibles à l'eau ou au moins en partie miscibles à l'eau, comme les polyols ; les mono-alcools inférieurs en C₂ à C₈, tels que l'éthanol et l'isopropanol. Par "température ambiante", il faut comprendre une température d'environ 25°C, à pression atmosphérique normale (760 mm de Hg).

Par "polyol", il faut comprendre toute molécule organique comportant au moins deux groupements hydroxyle libres. Comme polyols, on peut citer par exemple les glycols comme le butylène glycol, le propylène glycol, l'isoprène glycol, le glycérol et les polyéthylène glycols comme le PEG-8, le sorbitol, les sucres comme le glucose.

La phase aqueuse peut comprendre aussi tout additif habituel hydrosoluble ou hydrodispersible comme indiqué ci-après.

La phase aqueuse peut représenter de 50 à 98 % en poids, de préférence de 55 à 95 % en poids, et mieux de 60 à 90 % en poids en poids par rapport au poids total de la composition.

Le ou les composés miscibles à l'eau, tels que polyols et alcools inférieurs, peuvent être présents en une quantité allant de 0 à 30 % du poids total de la composition notamment de 0,1 à 30 % et mieux en une quantité allant de 1 à 20 %.

### Phase huileuse

La nature de la phase huileuse de l'émulsion selon l'invention n'est pas critique. La phase huileuse est une phase grasse comprenant au moins un corps gras choisi parmi les corps gras liquides à température ambiante (20-25°C) ou huiles, volatiles ou non, d'origine végétale, minérale ou synthétique, et leurs mélanges. Ces huiles sont physiologiquement acceptables.

Dans la présente invention, la quantité de phase huileuse n'inclut pas la quantité d'émulsionnants pouvant être utilisés selon l'invention.

La phase huileuse peut comprendre aussi tout additif habituel liposoluble ou lipodispersible comme indiqué ci-après.
Elle peut notamment comprendre d'autres corps gras tels que des cires, des composés pâteux, des alcools gras, des acides gras. La phase huileuse contient au moins une huile, plus particulièrement au moins une huile cosmétique. On entend par "huile" un corps gras liquide à la température ambiante (25°C).

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules R¹COOR² et R¹OR² dans laquelle R¹ représente le reste d'un acide gras ou d'un alcool gras comportant de 8 à 29 atomes de carbone, et R² représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam® ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; les polydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Selon un mode préféré de réalisation, la composition de l'invention comporte au moins une huile choisie parmi les huiles de silicone, les hydrocarbures linéaires ou ramifiés, les éthers et les esters de synthèse, et leurs mélanges, et notamment choisie parmi les huiles de silicone volatiles et les hydrocarbures ramifiés comme l'huile de Parléam®, et leurs mélanges.

La quantité de phase huileuse peut aller par exemple de 1 à 40% en poids, de préférence de 5 à 35 % en poids, de préférence de 10 à 35% en poids par rapport au poids total de la composition.
La quantité de phase huileuse dans la composition de l'invention est de préférence inférieure à 40% du poids total de la composition, de préférence inférieure ou égale à 35 % en poids, de préférence inférieure ou égale à 33% en poids.
Comme indiqué plus haut, cette quantité de phase huileuse ne comprend pas la quantité d'émulsionnant.

Selon un mode de réalisation, la composition selon l'invention comprend moins de 40% en poids d'huiles par rapport au poids total de la composition, en particulier moins de 35% en poids d'huiles.

### Polymère amphiphile additionnel

Selon un mode de réalisation, la composition selon l'invention comprend, outre le polymère amphiphile non réticulé, un polymère amphiphile réticulé, qui peut être en particulier choisi parmi les polymères réticulés d'AMPS modifiés hydrophobes.

Comme polymères réticulés d'AMPS modifiés hydrophobes, on peut utiliser notamment ceux comportant :
- 80 à 99 % en moles et de préférence de 85 à 99 % en moles de motifs acide 2-acrylamido 2-méthylpropane sulfonique (AMPS) de formule (I) 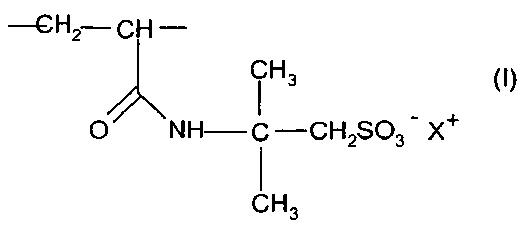
dans laquelle X⁺est un proton, un cation de métal alcalin, un cation alcalino-terreux, l'ion ammonium ou un cation organique ; et
- 1 à 20% en moles et de préférence de 1 à 15 % en moles de motifs hydrophobes de formule (II) suivante : 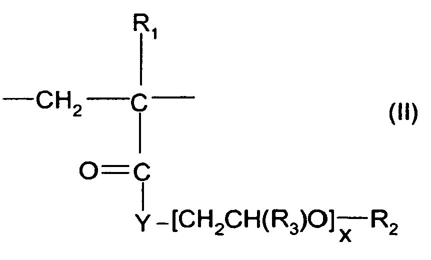
dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné comportant de 6 à 50 atomes de carbone, plus préférentiellement de 6 à 22 atomes de carbone, encore plus préférentiellement de 6 à 18 atomes de carbone et plus particulièrement de 12 à 18 atomes de carbone ; x désigne un nombre de moles d'oxyde d'alkylène et varie de 0 à 100, et mieux de 3 à 100.

Le radical R₂ est choisi de préférence parmi les radicaux alkyles en C₆-C₁₈ substantiellement linéaires (par exemple n-hexyle, n-octyle, n-décyle, n-hexadécyle, n-dodécyle ou lauryle, n-octadécyle, stéaryle ou béhényle), ramifiés ou cycliques (par exemple cyclododécane (C₁₂) ou adamantane (C₁₀)) ; les radicaux alkylperfluorés en C₆-C₁₈ (par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Parmi ces radicaux, on préfère plus particulièrement les radicaux alkyles linéaires et plus particulièrement les radicaux stéaryle et béhényle, et leurs mélanges.

Selon une forme particulièrement préférée de l'invention, le motif de formule (II) comporte au moins un motif oxyde d'alkylène (x ≥ 1) et de préférence plusieurs motifs oxyde d'alkylène (x > 1) constituant une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés (ou nombre de moles d'oxyde d'alkylène) varie en général de 3 à 100, plus préférentiellement de 3 à 50 et encore plus préférentiellement de 7 à 25.

De manière préféré, le polymère contient comme motif hydrophobe de formule (II), un motif de formule (III) : 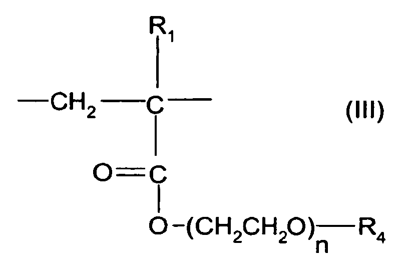 dans laquelle n désigne un nombre entier variant de 3 à 100, de préférence de 3 à 50 et plus préférentiellement de 7 à 25 ; R₁ est l'hydrogène ou un radical méthyle, et R₄ désigne un radical alkyle linéaire ou ramifié comportant de 6 à 22 atomes de carbone, de préférence de 10 à 22 atomes de carbone et mieux de 14 à 22 atomes de carbone.

Les polymères d'AMPS amphiphiles pouvant être utilisés dans la composition conforme à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH = 2,2-Azo-Bis-[2-Amidinopropane] Hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ces polymères modifiés hydrophobes peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La réaction peut être conduite à une température comprise entre 0 et 150°C, de préférence entre 10 et 100°C, soit à pression atmosphérique, soit sous pression réduite.
Elle peut aussi être réalisée sous atmosphère inerte, et de préférence sous azote.

Les polymères d'AMPS réticulés utilisés dans la composition de l'invention sont de préférence sous formes partiellement ou totalement neutralisées. On entend par « polymères partiellement neutralisés », des polymères qui sont neutralisés à au moins 90 %. Selon un mode préféré de réalisation de l'invention, ils sont totalement neutralisés.

De façon préférentielle, la neutralisation partielle ou totale des polymères d'AMPS utilisés conformément à l'invention est réalisée au moyen d'une base minérale (soude, potasse, ammoniaque) ou d'une base organique telle que la mono-, di- ou triéthanolamine, un aminométhylpropanediol, la N-méthyl-glucamine, les acides aminés basiques comme l'arginine et la lysine, et les mélanges de ces composés.

Les polymères utilisés sont réticulés. Les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

Parmi ces polymères, on peut citer :
- les copolymères d'AMPS réticulés décrits de manière générale dans le document EP-A-1,069,142 ;
- les copolymères réticulés, neutralisés ou non, comportant de 15 à 60 % en poids de motifs AMPS et de 40 à 85 % en poids de motifs (C₈-C₁₆)alkyl(méth)acrylamide ou de motifs (C₈-C₁₆)alkyl(méth)acrylate par rapport au polymère, tels que ceux décrits dans le document EP-A-750 899 ;
- les copolymères réticulés d'AMPS partiellement ou totalement neutralisés et de n-dodécylméthacrylamide, tels que ceux décrits dans les articles de Morishima cités ci-dessus.

Comme polymères plus particulièrement appropriés, on peut citer ceux obtenus par polymérisation d'acide 2-acrylamido-2-méthylpropane-sulfonique (AMPS) ou l'un de ses sels de sodium ou d'ammonium, avec un ester de l'acide méthacrylique ou acrylique, de préférence un ester de l'acide méthacrylique et d'un alcool oxyéthyléné de formule (IV) :

- CH = C(CH₃) - CO - O (CH₂CH₂O)n - R (IV)

dans laquelle n va de 7 à 25, et est de préférence égal à 8 ou à 25, et R est un reste alkyle ayant de 10 à 22 atomes de carbone.

Comme polymères de ce type, on peut citer plus spécialement celui commercialisé sous la dénomination ARISTOFLEX HMS par la société Clariant, qui est un copolymère AMPS/Méthacrylate de stéaryle éthoxylé (25 EO) réticulé, pour lequel dans la formule (IV) n est 25 et R est C₁₆-C₁₈, ou celui commercialisé sous la dénomination ARISTOFLEX HMB par la société Clariant, qui est un copolymère AMPS / Méthacrylate de béhényle éthoxylé (25 OE) réticulé, pour lequel dans la formule (IV) n est 25 et R est C22. On peut aussi utiliser un mélange de ces polymères.

La quantité de polymère amphiphile non réticulé dans la composition de l'invention peut aller par exemple, en matière active, de 0,1 à 5 % en poids, de préférence de 0,2 à 5 % en poids, mieux de 0,2 à 3 % en poids par rapport au poids total de la composition.

Selon un mode de réalisation avantageux, la composition selon l'invention comprend :
- un polymère amphiphile non réticulé choisi parmi les polymères préparés à partir de l'acide 2-acrylamido-2-méthylpropane-sulfonique (AMPS) ou l'un de ses sels de sodium ou d'ammonium, avec un ester de l'acide (méth)acrylique et d'un alcool en C10 à C20 oxyéthyléné comprenant de 6 à 25 groupes oxyéthylénés.
- un organopolysiloxane élastomère obtenu par réaction de diméthylpolysiloxane à terminaisons diméthylvinylsiloxy et de méthylhydrogénopolysiloxane à terminaisons triméthylsiloxy, en présence de catalyseur platine, un copolymère amphiphile, par exemple un copolymère donc le nom INCI est dimethicone/vinyldimethicone copolymer (ou polysilicone-11).

Selon un mode de réalisation, la composition ci-dessus peut comprendre en outre un polymère amphiphile réticulé qui peut être en particulier choisi parmi les polymères réticulés d'AMPS modifiés hydrophobes, comme par exemple un copolymère AMPS/Méthacrylate de stéaryle éthoxylé (25 EO) réticulé.

### Additifs

De façon connue, la composition pour application topique de l'invention peut contenir également un ou plusieurs des adjuvants habituels dans le domaine cosmétique ou dermatologique. Comme adjuvants, on peut citer par les gélifiants, les actifs, les conservateurs, les antioxydants, les parfums, les solvants, les sels, les charges, les filtres solaires (= filtres U.V.), les matières colorantes, les agents basiques (triéthanolamine, diéthanolamine, hydroxyde de sodium) ou acides (acide citrique), et encore les vésicules lipidiques ou tout autre type de vecteur (nanocapsules, microcapsules, etc...), et leurs mélanges. Ces adjuvants sont utilisés dans les proportions habituelles dans le domaine cosmétique, et par exemple de 0,01 à 30 % du poids total de la composition, et ils sont, selon leur nature, introduits dans la phase aqueuse de la composition ou dans la phase huileuse, ou encore dans des vésicules ou tout autre type de vecteur. Ces adjuvants ainsi que leurs concentrations doivent être tels qu'ils ne modifient pas la propriété recherchée pour l'émulsion de l'invention.

Selon la viscosité désirée pour la composition selon l'invention, on peut y incorporer un ou plusieurs gélifiant hydrophiles. Comme gélifiants hydrophiles, on peut citer par exemple les polymères carboxyvinyliques modifiés ou non, tels que les produits commercialisés sous les dénominations Carbopol (nom INCI : carbomer) par la société Noveon ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Clariant sous la dénomination « Hostacerin AMPS » (nom INCI : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin /Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de xanthane, la gomme guar, les alginates, les celluloses modifiées ou non ; et leurs mélanges. Quand ils sont présents, ces gélifiants doivent être introduits en quantité telle qu'ils ne modifient pas les propriétés de la composition selon l'invention. Comme gélifiants lipophiles, on peut citer notamment les argiles modifiées telles que le silicate de magnésium modifié (bentone gel VS38 de RHEOX), l'hectorite modifiée par le chlorure de distéaryl diméthyl ammonium (nom INCI : Disteardimonium hectorite) commercialisé sous la dénomination « bentone 38 CE » par la société RHEOX.

Le gélifiant peut être présente en une teneur en matière active allant de 0,05% à 10% en poids et préférentiellement de 0,1% à 5% en poids par rapport au poids total de la composition.

Comme charges qui peuvent être utilisées dans la composition de l'invention, on peut citer par exemple, les pigments tels que les oxydes de titane, de zinc ou de fer et les pigments organiques ; le kaolin ; la silice ; le talc ; le nitrure de bore ; les poudres sphériques organiques, les fibres ; et leurs mélanges. Comme poudres sphériques organiques, on peut citer par exemple les poudres de polyamide et notamment les poudres de Nylon® telles que Nylon-1 ou Polyamide 12, commercialisées sous les dénominations ORGASOL par la société Atochem ; les poudres de polyéthylène ; le Téflon® ; les microsphères à base de copolymères acryliques, telles que celles en copolymère diméthacrylate d'éthylène glycol/ methacrylate de lauryle commercialisées par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch. Comme fibres, on peut citer par exemple les fibres de polyamide, telles que notamment les fibres de Nylon 6 (ou Polyamide 6) (nom INCI : Nylon 6), de Nylon 6,6 (ou Polyamide 66) (Nom INCI : Nylon 66) ou telles que les fibres de poly-p-phénylène téréphtamide ; et leurs mélanges. Ces charges peuvent être présentes dans des quantités allant de 0 à 20 % en poids et de préférence de 0,5 à 10 % en poids par rapport au poids total de la composition.

Comme actifs utilisables dans la composition de l'invention, on peut citer par exemple, les agents hydratants tels que les hydrolysats de protéines ; le hyaluronate de sodium ; les polyols comme la glycérine, les glycols comme les polyéthylène glycols, et les dérivés de sucre ; les anti-inflammatoires ; les oligomères procyannidoliques ; les vitamines comme la vitamine A (rétinol), la vitamine E (tocophérol), la vitamine K, la vitamine C (acide ascorbique), la vitamine B5 (panthénol), la vitamine B3 ou PP (niacinamide), les dérivés de ces vitamines (notamment esters) et leurs mélanges ; les agents kératolytiques et/ou desquamants tels que l'acide salicylique et ses dérivés, les alpha-hydroxyacides comme l'acide lactique et l'acide glycolique et leurs dérivés, l'acide ascorbique et ses dérivés ; l'urée ; la caféine ; les dépigmentants tels que l'acide kojique, l'hydroquinone et l'acide caféique; l'acide salicylique et ses dérivés ; les rétinoïdes tels que les caroténoïdes et les dérivés de vitamine A ; l'hydrocortisone; la mélatonine ; les extraits d'algues, de champignons, de végétaux, de levures, de bactéries ; les stéroïdes ; les actifs anti-bactériens comme le 2,4,4'-trichloro-2'-hydroxy diphényl éther (ou triclosan), le 3,4,4'-trichlorocarbanilide (ou triclocarban) et les acides indiqués ci-dessus et notamment l'acide salicylique et ses dérivés ; les enzymes ; les flavonoïdes ; les agents tenseurs tels que les polymères synthétiques, les protéines végétales, les polysaccharides d'origine végétale sous forme ou non de microgels, les amidons, les dispersions de cires, les silicates mixtes et les particules colloïdales de charges inorganiques ; les céramides ; les agents anti-inflammatoires ; les agents apaisants ; les agents matifiants ; les agents anti-chute et/ou repousse des cheveux ; les agents anti-rides ; les huiles essentielles ; et leurs mélanges ; et tout actif approprié pour le but final de la composition.

Les filtres U.V. peuvent être organiques ou minéraux (ou filtres U.V. physiques). Ils peuvent être présents en une quantité en matière active allant de 0,01 à 20 % en poids de matière active, de préférence de 0,1 à 15 % en poids, et mieux 0,2 à 10 % en poids par rapport au poids total de la composition.

Comme exemples de filtres organiques, actifs dans l'UV-A et/ou l'UV-B, pouvant être ajoutés dans la composition de l'invention, on peut citer par exemple, les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone ; les dérivés de β ,β'-diphénylacrylate, les dérivés de benzotriazole, les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665; les dimères dérivés d'α-alkylstyrène tels que ceux décrits dans la demande de brevet DE19855649.

La quantité totale de filtres UV organiques dans les compositions selon l'invention peut aller par exemple de 0,1 à 20% en poids par rapport au poids total de la composition, et de préférence allant de 0,2 à 15% en poids par rapport au poids total de la composition.

Comme filtres physiques pouvant être ajoutés dans la composition de l'invention, on peut citer par exemple les pigments et nanopigments d'oxydes métalliques, enrobés ou non enrobés, notamment les oxydes de titane, de fer, de zirconium, de zinc ou de cérium, et leurs mélanges, ces oxydes pouvant être sous forme de micro- ou nanoparticules (nanopigments), éventuellement enrobées.

Les compositions de l'invention sont avantageusement préparées selon un procédé dans lequel la phase grasse huileuse, comprenant les huiles et éventuellement les autres corps gras, est émulsionnée dans la phase aqueuse (dans laquelle a été introduite le polymère amphiphile), sous agitation.

Selon un mode de réalisation, les compositions de l'invention sont préparées selon un procédé dans lequel la phase grasse huileuse, comprenant les huiles et éventuellement les autres corps gras, est émulsionnée dans la phase aqueuse (dans laquelle a été introduite le polymère amphiphile), sous faible agitation, c'est-à-dire sous un faible degré de cisaillement.
L'agitation se fait de préférence avec un barreau aimanté ou tout autre système d'agitation donnant une faible agitation et donc de faible énergie, à une température pouvant aller de 20° à 45°C. On entend par « faible agitation », une agitation faite à un degré de cisaillement inférieur à 1000 s⁻¹
Ainsi le procédé d'émulsification sous faible cisaillement peut se faire avec tout autre système d'agitation donnant une faible agitation et donc de faible énergie comme par exemple :
- à l'aide d'une pâle ou hélice,
- en cuve munie d'une turbine de fond de cuve, d'une pale raclante, ou d'une pale de mélange centrale contrarotative et un chauffage/refroidissement par la double enveloppe de la cuve. On peut citer comme exemples les cuves Macef et Maxilab de la société OLSA, les cuves proposées par la société Pierre Guérin,
- à l'aide d'un moulin colloïdal,
- à l'aide d'un émulseur statique,
- avec une turbine en ligne, de la marque IKA ou KMF par exemple.

Les compositions selon l'invention peuvent se présenter par exemple sous toutes les formes galéniques des émulsions H/E, par exemple sous forme de sérum, de lait ou de crème, et elles sont préparées selon les méthodes usuelles. Les compositions, objets de l'invention, sont destinées à une application topique et peuvent constituer notamment une composition dermatologique ou cosmétique, par exemple destinée au soin (anti-rides, anti-âge, hydratation, protection solaire, etc), au traitement, au nettoyage et au maquillage des matières kératiniques et notamment de la peau, des lèvres, des cheveux, des cils, des cheveux et des ongles des êtres humains.

Selon un mode préféré de réalisation de l'invention, la composition constitue une composition cosmétique et est destinée à une application topique sur la peau.

Les exemples qui suivent permettront de mieux comprendre l'invention, sans toutefois présenter un caractère limitatif. Les quantités indiquées sont en % en poids, sauf mention contraire.

### EXEMPLES

### Exemples 1 et 2 :

On prépare les émulsions suivantes :

| | **Emulsion 1 (selon l'invention)** | **Emulsion 2 (comparatif)** |
|---|---|---|
| Copolymère d'AMPS et de méthacrylate de Genapol T-080 (taux de greffage = 7,35%) (Aristoflex SNC de Clariant) à 92% en matières actives dans mélange eau/ alcool | 0,4% | - |
| Acide poly-2-acrylamido-2-méthylpropane sulfonique réticulé et partiellement neutralisé par l'ammoniaque à 97,4% en matières actives dans mélange alcool. (Hostacerin AMPS de Hoechst) | 0,6% | 1% |
| Mélange de polydimethylsiloxane alpha-omega dihydroxylé et polydimethylsiloxane 5cSt (12/88) (DC1503 Fluid de Dow Corning) | 2% | 2% |
| Mélange d'organopolysiloxane réticulé et de cyclohexasiloxane (10/90) (Gransil RPS-6 de GRANT Industries) | 25% | 25% |
| Poudre de polytetrafluoroéthylène ( 12 microns) (Fuoropure 812C de Shamrock) | 1,5% | 1,5% |
| Particules concaves siliconées (diamètre : 2,5µm) (NLK 506 de Takemoto Oil & Fat) | 1,5% | 1,5% |
| Glycérine | 7% | 7% |
| Tetraoctanoate de pentaerythrityle | 2% | 2% |
| Mica enrobé TiO2 | 0,5% | 0,5% |
| EDTA | 0,1% | 0,1% |
| Conservateurs | qs | qs |
| Ethanol | 5% | 5% |
| Eau | Qsp 100 | Qsp 100 |

### Mode opératoire :

Le copolymère amphiphile non réticulé, fourni sous forme de poudre, est solubilisé dans la phase aqueuse (comprenant l'eau et la glycérine) pendant 30 minutes sous agitation à 80°C ; la solution obtenue est macroscopiquement homogène.
Pour l'émulsion 1, on ajoute ensuite le copolymère amphiphile réticulé (Hostacerin AMPS) que l'on laisse gonfler pendant 30 minutes sous agitation à 80°C puis on laisse refroidir le gel obtenu.
Parallèlement, on mélange au Rayneri la polydimethylsiloxane alpha-omega dihydroxylé, les conservateurs, le tetraoctanoate de pentaerythrityle, l'élastomère siliconé puis les charges. Puis on introduit ce mélange dans le gel aqueux et on homogénéise l'ensemble à l'aide d'une Turbine en ligne IKA à 300 t/min pendant 10 minutes. On ajoute l'éthanol et on homogénéise à nouveau l'ensemble sous l'aide d'une Turbine en ligne IKA à 300 t/min pendant 10 minutes.

On a évalué visuellement l'aspect macroscopique et l'aspect microscopique de chaque composition selon la méthode indiquée plus haut, ainsi que la transmittance :

| | **Emulsion 1** | **Emulsion 2** |
|---|---|---|
| **Aspect visuel** | lisse | peu lisse |
| **Aspect microscopique Taille moyenne des globules huileux** | environ 150 µm | dispersion grossière et hétérogène des plages huileuses impossibilité de mesurer une taille de globules |
| **Transmittance** | 46,35% | 38,55 % |

Ces exemples démontrent que la présence d'un polymère amphiphile non réticulé est essentielle pour l'obtention d'un émulsion stable présentant des globules huileux bien définis et de taille importante. En outre l'émulsion 1 selon l'invention présente une transmittance plus élevée que l'émulsion 2 et est donc plus translucide que cette dernière.

### Exemples 3 et 4:

On prépare les émulsions suivantes :

| | **Emulsion 3 (selon l'invention)** | **Emulsion 4 (selon l'invention)** |
|---|---|---|
| Copolymère d'AMPS et de méthacrylate de Genapol T-080 (taux de greffage = 7,35%) (Aristoflex SNC de Clariant) à 92% en matières actives dans mélange eau/ alcool | 0,6% | 0,45% |
| Acide poly-2-acrylamido-2-méthylpropane sulfonique réticulé et partiellement neutralisé par l'ammoniaque à 97,4% en matières actives dans mélange alcool. (Hostacerin AMPS de Hoechst) | 0,6% | 0,65% |
| Mélange de polydimethylsiloxane alpha-omega dihydoxylé et polydimethylsiloxane 5cSt (12/88) (DC1503 Fluid de Dow Corning) | 2% | 2% |
| Mélange d'organopolysiloxane réticulé et de cyclopentasiloxane (10/90) (Gransil RPS de GRANT Industries) | 25% | - |
| Mélange d'organopolysiloxane réticulé et de cyclohexasiloxane (10/90) (Gransil RPS-6 de GRANT Industries) | - | 25% |
| Caprylic capric triglycerides/Stearalkonium hectorite/Propylene carbonate (84/12/4) Myglyol gel B de Sasol | - | 1,5% |
| Poudre de polytetrafluoroéthylène ( 12 microns) (Fuoropure 812C de Shamrock) | 1,5% | 1,5% |
| Particules concaves siliconées (diamètre : 2,5µm) (NLK 506 de Takemoto Oil & Fat) | 1,5% | 1,5% |
| Glycérine | 7% | 13% |
| Tetraoctanoate de pentaerythrityle | 2% | 4% |
| Mica enrobé TiO2 | 0,5% | 0,5% |
| Actifs hydrosolubles | - | 10% |
| Actifs liposolubles | - | 1 |
| EDTA | 0,1% | 0,1% |
| Parfum | -% | 0,11% |
| Conservateurs | qs | qs |
| Ethanol | 5% | 5% |
| Eau | Qsp 100 | Qsp 100 |

Ces émulsions ont été jugées comme présentant un aspect transparent, nacré et lisse par un panel d'expertes.

## Revendications

1. Composition pour application topique, sous forme d'émulsion huile-dans-eau comprenant une phase huileuse dispersée dans une phase aqueuse, **caractérisée en ce qu'**elle comprend
- au moins un polymère amphiphile non réticulé comprenant :
(a) de 80 à 99% en moles de motif acide 2-acrylamido 2-méthylpropane sulfonique (AMPS) de formule (3) suivante : 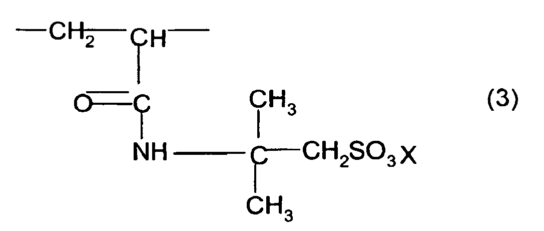 dans laquelle X est un proton, un cation de métal alcalin, un cation alcalino-terreux ou l'ion ammonium ; étant entendu que lorsque X représente un cation métallique alcalino terreux, il partage 2 charges positives avec 2 groupements SO3
(b) et de 1 à 20% en moles, et de préférence de 1 à 15% en moles de motif de formule (3 bis) suivante : 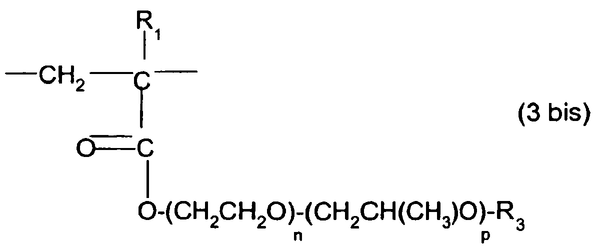 dans laquelle n et p, indépendamment l'un de l'autre désignent un nombre de moles et varie de 0 à 30, de préférence de 1 à 20 sous réserve que n + p soit inférieur ou égal à 30, de préférence inférieur à 25 et encore mieux inférieur à 20 ; R₁ désigne un atome d'hydrogène, un radical alkyle linéaire ou ramifié en C₁-C₆ (de préférence méthyle) et R₃ désigne un alkyle linéaire ou ramifié comportant m atomes de carbone allant de 6 à 30, de préférence de 10 à 25 atomes de carbone, et
- au moins un organopolysiloxane élastomère.

2. Composition selon la revendication 1, **caractérisée en ce que** le polymère amphiphile a une masse molaire en poids allant de 10 000 à 10 000 000.

3. Composition selon l'une quelconque des revendications précédentes, **caractérisée en ce que** la quantité (en matière active) de polymère amphiphile va 0,01% à 10% en poids, de préférence de 0,05% à 5% en poids et encore mieux de 0,1 à 3% en poids par rapport au poids total de la composition.

4. Composition selon l'une quelconque des revendications précédentes, **caractérisée en ce que** le polymère amphiphile est neutralisé partiellement ou totalement par une base minérale ou organique.

5. Composition selon l'une quelconque des revendications précédentes, **caractérisée en ce que** le polymère amphiphile est choisi parmi les polymères préparés à partir de l'acide 2-acrylamido-2-méthylpropane-sulfonique (AMPS) ou l'un de ses sels de sodium ou d'ammonium, avec un ester d'acide (méth)acrylique et :
- d'un alcool en C₁₀-C₁₈ oxyéthyléné par 8 moles d'oxyde d'éthylène (Genapol C-080 de la société Clariant),
- d'un alcool oxo en C₁₁ oxyéthyléné par 8 moles d'oxyde d'éthylène ,
- d'un alcool oxo en C₁₁ oxyéthyléné par 7 moles d'oxyde d'éthylène ,
- d'un alcool en C₁₂-C₁₄oxyéthyléné par 7 moles d'oxyde d'éthylène,
- d'un alcool en C₁₂-C₁₄oxyéthyléné par 9 moles d'oxyde d'éthylène,
- d'un alcool en C₁₂-C₁₄ oxyéthyléné par 11 moles d'oxyde d'éthylène,
- d'un alcool en C₁₆-C₁₈ oxyéthyléné par 8 moles d'oxyde d'éthylène,
- d'un alcool en C₁₆-C₁₈ oxyéthyléné par 11 moles d'oxyde d'éthylène,
- d'un alcool en C₁₆-C₁₈ oxyéthyléné par 15 moles d'oxyde d'éthylène,
- d'un alcool en C₁₆-C₁₈ oxyéthyléné par 20 moles d'oxyde d'éthylène,
- d'un alcool en C₁₆-C₁₈ oxyéthyléné par 25 moles d'oxyde d'éthylène,
- d'un alcool en C₁₈-C₂₂ oxyéthyléné par 25 moles d'oxyde d'éthylène,
- d'un alcool en iso-C₁₆-C₁₈ oxyéthyléné par 25 moles d'oxyde d'éthylène.

6. Composition selon l'une quelconque des revendications précédentes, **caractérisée en ce que** le polymère amphiphile est un copolymère d'AMPS et de méthacrylate d'alcool en C₁₆-C₁₈ comportant de 6 à 25 groupes oxyéthylénés, obtenu à partir d'acide méthacrylique ou d'un sel d'acide méthacrylique et d'un alcool en C₁₆-C₁₈ oxyéthyléné par 6 à 25 moles d'oxyde d'éthylène ou un colymère d'AMPS et de méthacrylate d'alcool en C₁₂-C₁₄ comportant de 6 à 25 groupes oxyéthylénés, obtenus à partir d'acide méthacrylique ou d'un sel d'acide méthacrylique et d'un alcool en C₁₂-C₁₄ oxyéthyléné par 6 à 25 moles d'oxyde d'éthylène.

7. Composition selon l'une quelconque des revendications précédentes, **caractérisée en ce que** l'organopolysiloxane élastomère est choisi parmi ceux obtenus :
- par réaction d'addition réticulation de diorganosiloxane contenant au moins un hydrogène lié au silicium et d'un polyoxyalkylène ayant au moins deux groupements à insaturation éthylénique;
- par réaction d'addition réticulation de diorganosiloxane contenant au moins un hydrogène lié au silicium et de composés polyglycérolés ayant des groupements à insaturation éthylénique, notamment en présence de catalyseur platine ;
- par réaction d'addition réticulation de diorganosiloxane contenant au moins un hydrogène lié au silicium et de diorganopolysiloxane ayant des groupements à insaturation éthylénique liés au silicium ;
- par réaction de condensation réticulation déhydrogénation entre un diorganopolysiloxane à terminaisons hydroxyle et un diorganopolysiloxane contenant au moins un hydrogène lié au silicium ;
- par réaction de condensation réticulation d'un diorganopolysiloxane à terminaisons hydroxyle et d'un organopolysilane hydrolysable ;
- par réticulation thermique d'organopolysiloxane ;
- par réticulation d'organopolysiloxane par radiations de haute énergie.

8. Composition selon l'une quelconque des revendications précédentes, **caractérisée en ce que** l'organopolysiloxane élastomère est obtenu par réaction d'addition réticulation (A) de diorganopolysiloxane contenant au moins deux atomes d'hydrogènes liés chacun à un atome de silicium différent, et (B) de diorganopolysiloxane ayant au moins deux groupements à insaturation éthylénique liés au silicium, notamment en présence (C) de catalyseur platine.

9. Composition selon l'une quelconque des revendications précédentes, **caractérisée en ce que** l'organopolysiloxane élastomère est obtenu par réaction de diméthylpolysiloxane à terminaisons diméthylvinylsiloxy et de méthylhydrogénopolysiloxane à terminaisons triméthylsiloxy, en présence de catalyseur platine.

10. Composition selon l'une quelconque des revendications précédentes, **caractérisée en ce que** l'organopolysiloxane élastomère est un copolymère dimethicone/vinyldimethicone copolymer (ou polysilicone-11).

11. Composition selon l'une quelconque des revendications précédentes, **caractérisée en ce qu'**elle comprend un polymère amphiphile réticulé.

12. Composition selon l'une des revendications précédentes, **caractérisée en ce que** la quantité en matières actives d'organopolysiloxane(s) élastomère(s) va de 0,01 à 10 % en poids par rapport au poids total de la composition, et de préférence de 0,5 à 7 % en poids et mieux de 1 à 4% par rapport au poids total de la composition.

13. Composition selon l'une quelconque des revendications précédentes, **caractérisée en ce que** la quantité de phase huileuse est inférieure à 40 % du poids total de la composition, de préférence inférieure ou égale à 35% en poids.

14. Composition selon l'une quelconque des revendications précédentes, **caractérisée en ce que** l'émulsion présentent une taille moyenne des globules huileux allant de 15 à 500 microns, de préférence de 15 à 300 µm et encore mieux de 15 à 250 µm.

15. Composition selon l'une quelconque des revendications précédentes, **caractérisée en ce que** qu'elle comprend un taux d'émulsionnant inférieur ou égal à 1% en poids par rapport au poids de la composition, de préférence inférieur ou égal à 0,5% en poids et mieux inférieur ou égal à 0,1% en poids.

16. Procédé de traitement cosmétique des matières kératiniques, **caractérisé en ce qu'**on applique sur les matières kératiniques, une composition cosmétique selon l'une quelconque des revendications 1 à 15.

## Patentansprüche

1. Zusammensetzung zur topischen Applikation in Form einer Öl-in-Wasser-Emulsion, die eine in einer wässrigen Phase dispergierte ölige Phase umfasst, **dadurch gekennzeichnet, dass** sie
- mindestens ein unvernetztes amphiphiles Polymer, umfassend:
(a) 80 bis 99 Mol-% 2-Acrylamido-2-methyl-propansulfonsäure-Einheiten (AMPS-Einheiten) der folgenden Formel (3): 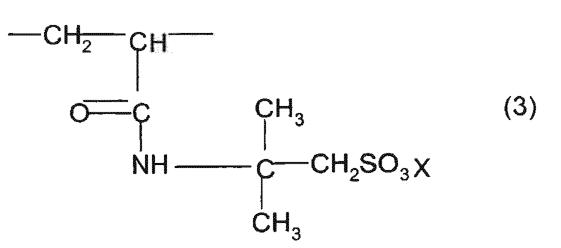 in der X für ein Proton, ein Alkalimetallkation, ein Erdalkalimetallkation oder ein Ammoniumion steht; mit der Maßgabe, dass X dann, wenn es für ein Erdalkalimetallkation steht, mit 2 SO3-Gruppen 2 positive Ladungen teilt;
(b) und 1 bis 20 Mol-% und vorzugsweise 1 bis 15 Mol-% Einheiten der folgenden Formel (3a): 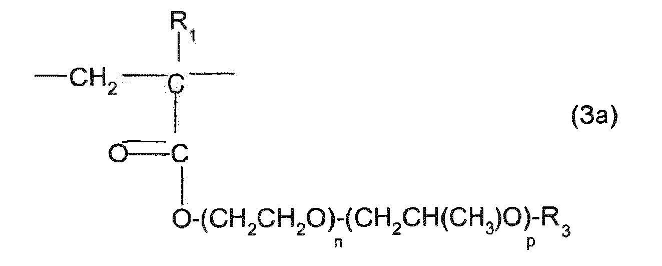 in der n und p unabhängig voneinander für eine Molzahl stehen und von 0 bis 30 und vorzugsweise von 1 bis 20 variieren unter der Bedingung, dass n + p kleiner gleich 30, vorzugsweise kleiner als 25 und noch besser kleiner als 20 ist; R₁ für ein Wasserstoffatom oder einen linearen oder verzweigten C₁-C₆-Alkylrest (vorzugsweise Methylrest) steht und R₃ für ein lineares oder verzweigtes Alkyl mit m Kohlenstoffatomen im Bereich von 6 bis 30 und vorzugweise 10 bis 25 Kohlenstoffatomen steht, und
- mindestens ein elastomeres Organopolysiloxan umfasst.

2. Zusammensetzung nach Anspruch 1, **dadurch gekennzeichnet, dass** das amphiphile Polymer ein gewichtsmittleres Molekulargewicht im Bereich von 10.000 bis 10.000.000 aufweist.

3. Zusammensetzung nach einem der vorhergehenden Ansprüche, **dadurch gekennzeichnet, dass** die Menge (als Aktivsubstanz) an amphiphilem Polymer 0,01 bis 10 Gew.-%, vorzugsweise 0,05 bis 5 Gew.-% und noch besser 0,1 bis 3 Gew.-%, bezogen auf das Gesamtgewicht der Zusammensetzung, beträgt.

4. Zusammensetzung nach einem der vorhergehenden Ansprüche, **dadurch gekennzeichnet, dass** das amphiphile Polymer teilweise oder vollständig durch eine anorganische oder organische Base neutralisiert ist.

5. Zusammensetzung nach einem der vorhergehenden Ansprüche, **dadurch gekennzeichnet, dass** das amphiphile Polymer aus den aus 2-Acrylamido-2-methylpropansulfonsäure (AMPS) oder einem ihrer Natrium- oder Ammoniumsalze mit einem Ester von (Meth)acrylsäure und:
- einem mit 8 mol Ethylenoxid oxyethylenierten C₁₀-C₁₈-Alkohol (Genapol C-080 von der Firma Clariant),
- einem mit 8 mol Ethylenoxid oxyethylenierten C₁₁-Oxoalkohol,
- einem mit 7 mol Ethylenoxid oxyethylenierten C₁₁-Oxoalkohol,
- einem mit 7 mol Ethylenoxid oxyethylenierten C₁₂-C₁₄-Alkohol,
- einem mit 9 mol Ethylenoxid oxyethylenierten C₁₂-C₁₄-Alkohol,
- einem mit 11 mol Ethylenoxid oxyethylenierten C₁₂-C₁₄-Alkohol,
- einem mit 8 mol Ethylenoxid oxyethylenierten C₁₆-C₁₈-Alkohol,
- einem mit 11 mol Ethylenoxid oxyethylenierten C₁₆-C₁₈-Alkohol,
- einem mit 15 mol Ethylenoxid oxyethylenierten C₁₆-C₁₈-Alkohol,
- einem mit 20 mol Ethylenoxid oxyethylenierten C₁₆-C₁₈-Alkohol,
- einem mit 25 mol Ethylenoxid oxyethylenierten C₁₆-C₁₈-Alkohol,
- einem mit 25 mol Ethylenoxid oxyethylenierten C₁₈-C₂₂-Alkohol,
- einem mit 25 mol Ethylenoxid oxyethylenierten C₁₆-C₁₈-Isoalkohol,
hergestellten Polymeren ausgewählt ist.

6. Zusammensetzung nach einem der vorhergehenden Ansprüche, **dadurch gekennzeichnet, dass** es sich bei dem amphiphilen Polymer um ein Copolymer von AMPS und Methacrylat von C₁₆-C₁₈-Alkohol mit 6 bis 25 Oxyethylengruppen, erhalten aus Methacrylsäure oder einem Methacrylsäuresalz und einem mit 6 bis 25 mol Ethylenoxid oxyethylenierten C₁₆-C₁₈-Alkohol, oder ein Copolymer von AMPS und Methacrylat von C₁₂-C₁₄-Alkohol mit 6 bis 25 Oxyethylengruppen, erhalten aus Methacrylsäure oder einem Methacrylsäuresalz und einem mit 6 bis 25 mol Ethylenoxid oxyethylenierten C₁₂-C₁₄-Alkohol, handelt.

7. Zusammensetzung nach einem der vorhergehenden Ansprüche, **dadurch gekennzeichnet, dass** das elastomere Organopolysiloxan aus denjenigen ausgewählt ist, die
- durch Vernetzungsadditionsreaktion von Diorganosiloxan mit mindestens einem siliciumgebundenen Wasserstoff und einem Polyoxyalkylen mit mindestens zwei ethylenisch ungesättigten Gruppen,
- durch Vernetzungsadditionsreaktion von Diorganosiloxan mit mindestens einem siliciumgebundenen Wasserstoff und Polyglycerinverbindungen mit ethylenisch ungesättigten Gruppen, insbesondere in Gegenwart von Platinkatalysator;
- durch Vernetzungsadditionsreaktion von Diorganosiloxan mit mindestens einem siliciumgebundenen Wasserstoff und Diorganopolysiloxan mit siliciumgebundenen ethylenisch ungesättigten Gruppen;
- durch Dehydrierungsvernetzungskondensationsreaktion zwischen einem Diorganopolysiloxan mit Hydroxylendgruppen und einem Diorganopolysiloxan mit mindestens einem siliciumgebundenen Wasserstoff,
- durch Vernetzungskondensationsreaktion eines Diorganopolysiloxans mit Hydroxylendgruppen und eines hydrolysierbaren Organopolysilans,
- durch thermische Vernetzung von Organopolysiloxan oder
- durch Vernetzung von Organopolysiloxan durch energiereiche Strahlung
erhalten werden.

8. Zusammensetzung nach einem der vorhergehenden Ansprüche, **dadurch gekennzeichnet, dass** das elastomere Organopolysiloxan durch Vernetzungsadditionsreaktion (A) von Diorganopolysiloxan mit mindestens zwei Wasserstoffatomen, jeweils an ein anderes Siliciumatom gebunden sind, und (B) Diorganopolysiloxan mit mindestens zwei siliciumgebundenen ethylenisch ungesättigten Gruppen, insbesondere in Gegenwart (C) von Platinkatalysator, erhalten wird.

9. Zusammensetzung nach einem der vorhergehenden Ansprüche, **dadurch gekennzeichnet, dass** das elastomere Organopolysiloxan durch Reaktion von Dimethylpolysiloxan mit Dimethylvinylsiloxy-Endgruppen und Methylhydrogenopolysiloxan mit Trimethylsiloxy-Endgruppen in Gegenwart von Platinkatalysator erhalten wird.

10. Zusammensetzung nach einem der vorhergehenden Ansprüche, **dadurch gekennzeichnet, dass** es sich bei dem elastomeren Organopolysiloxan um ein Dimethicon/Vinyldimethicon-Copolymer (oder Polysilikon-11) handelt.

11. Zusammensetzung nach einem der vorhergehenden Ansprüche, **dadurch gekennzeichnet, dass** sie ein vernetztes amphiphiles Polymer umfasst.

12. Zusammensetzung nach einem der vorhergehenden Ansprüche, **dadurch gekennzeichnet, dass** die Aktivsubstanzmenge von elastomerem Organopolysiloxan im Bereich von 0,01 bis 10 Gew.-%, bezogen auf das Gesamtgewicht der Zusammensetzung, und vorzugsweise von 0,5 bis 7 Gew.-% und noch besser von 1 bis 4 Gew.-%, bezogen auf das Gesamtgewicht der Zusammensetzung, liegt.

13. Zusammensetzung nach einem der vorhergehenden Ansprüche, **dadurch gekennzeichnet, dass** die Menge an öliger Phase kleiner als 40% des Gesamtgewichts der Zusammensetzung und vorzugsweise kleiner gleich 35 Gew.-% ist.

14. Zusammensetzung nach einem der vorhergehenden Ansprüche, **dadurch gekennzeichnet, dass** die Emulsion eine mittlere Öltröpfchengröße im Bereich von 15 bis 500 Mikron, vorzugsweise 15 bis 300 µm und noch besser 15 bis 250 µm aufweist.

15. Zusammensetzung nach einem der vorhergehenden Ansprüche, **dadurch gekennzeichnet, dass** sie einen Emulgatoranteil kleiner gleich 1 Gew.-%, bezogen auf das Gewicht der Zusammensetzung, vorzugsweise kleiner gleich 0,5 Gew.-% und noch besser kleiner gleich 0,1 Gew.-% aufweist.

16. Verfahren zur kosmetischen Behandlung von Keratinmaterialien, **dadurch gekennzeichnet, dass** man auf die Keratinmaterialien eine kosmetische Zusammensetzung nach einem der Ansprüche 1 bis 15 aufbringt.

## Claims

1. Composition for topical application, in the form of an oil-in-water emulsion comprising an oily phase dispersed in an aqueous phase, **characterized in that** it comprises:
- at least one uncrosslinked amphiphilic polymer comprising:
(a) from 80 to 99 mol% of 2-acrylamido-2-methylpropanesulphonic acid (AMPS) units of formula (3) below: 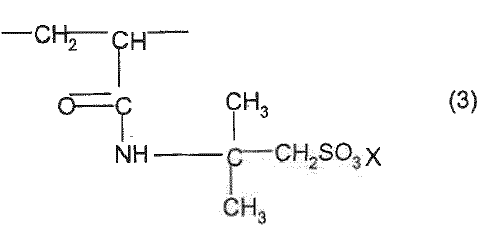 in which X is a proton, an alkali metal cation, an alkaline-earth metal cation or an ammonium ion; it may be understood that when X represents an alkaline-earth metal cation, it shares two positive charges with two SO₃ groups;
(b) and from 1 to 20 mol%, and preferably from 1 to 15 mol%, of units of formula (3a) below: 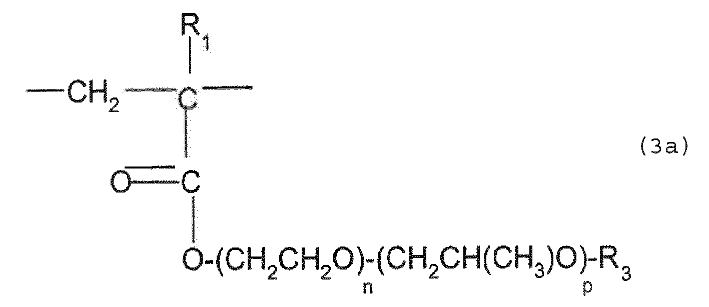 in which n and p, independently of one another, denote a number of moles and vary from 0 to 30, preferably from 1 to 20 on condition that n + p is less than or equal to 30, preferably less than 25 and better still less than 20; R₁ denotes a hydrogen atom, or a linear or branched C₁-C₆ alkyl (preferably methyl) radical and R₃ denotes a linear or branched alkyl group comprising m carbon atoms ranging from 6 to 30, preferably from 10 to 25 carbon atoms; and
- at least one organopolysiloxane elastomer.

2. Composition according to Claim 1, **characterized in that** the amphiphilic polymer has a weight-average molecular weight ranging from 10 000 to 10 000 000.

3. Composition according to either one of the preceding claims, **characterized in that** the amount (as active material) of amphiphilic polymer ranges from 0.01% to 10% by weight, preferably from 0.05% to 5% by weight and better still from 0.1 to 3% by weight relative to the total weight of the composition.

4. Composition according to any one of the preceding claims, **characterized in that** the amphiphilic polymer is partially or completely neutralized by a mineral or organic base.

5. Composition according to any one of the preceding claims, **characterized in that** the amphiphilic polymer is chosen from polymers prepared from 2-acrylamido-2-methylpropanesulphonic acid (AMPS) or one of its sodium or ammonium salts, with an ester of (meth)acrylic acid and:
- of a C₁₀-C₁₈ alcohol oxyethylenated with 8 mol of ethylene oxide (Genapol C-080 from Clariant);
- of a C₁₁ oxo alcohol oxyethylenated with 8 mol of ethylene oxide;
- of a C₁₁ oxo alcohol oxyethylenated with 7 mol of ethylene oxide;
- of a C₁₂-C₁₄ alcohol oxyethylenated with 7 mol of ethylene oxide;
- of a C₁₂-C₁₄ alcohol oxyethylenated with 9 mol of ethylene oxide;
- of a C₁₂-C₁₄ alcohol oxyethylenated with 11 mol of ethylene oxide;
- of a C₁₆-C₁₈ alcohol oxyethylenated with 8 mol of ethylene oxide;
- of a C₁₆-C₁₈ alcohol oxyethylenated with 11 mol of ethylene oxide;
- of a C₁₆-C₁₈ alcohol oxyethylenated with 15 mol of ethylene oxide;
- of a C₁₆-C₁₈ alcohol oxyethylenated with 20 mol of ethylene oxide;
- of a C₁₆-C₁₈ alcohol oxyethylenated with 25 mol of ethylene oxide;
- of a C₁₈-C₂₂ alcohol oxyethylenated with 25 mol of ethylene oxide;
- of a C₁₆-C₁₈ iso alcohol oxyethylenated with 25 mol of ethylene oxide.

6. Composition according to any one of the preceding claims, **characterized in that** the amphiphilic polymer is a copolymer of AMPS and of a methacrylate of a C₁₆-C₁₈ alcohol comprising from 6 to 25 oxyethylenated groups, obtained from methacrylic acid or from a methacrylic acid salt and from a C₁₆-C₁₈ alcohol oxyethylenated with 6 to 25 mol of ethylene oxide or a copolymer of AMPS and of a methacrylate of a C₁₂-C₁₄ alcohol comprising from 6 to 25 oxyethylenated groups, obtained from methacrylic acid or from a methacrylic acid salt and from a C₁₂-C₁₄ alcohol oxyethylenated with 6 to 25 mol of ethylene oxide.

7. Composition according to any one of the preceding claims, **characterized in that** the organopolysiloxane elastomer is chosen from those obtained:
- by crosslinking addition reaction of a diorganosiloxane containing at least one hydrogen bonded to the silicon and of a polyoxyalkylene having at least two ethylenically unsaturated groups;
- by crosslinking addition reaction of a diorganosiloxane containing at least one hydrogen bonded to the silicon and of polyglycerolated compounds having ethylenically unsaturated groups, especially in the presence of a platinum catalyst;
- by crosslinking addition reaction of a diorganosiloxane containing at least one hydrogen bonded to the silicon and of a diorganopolysiloxane having ethylenically unsaturated groups bonded to the silicon;
- by dehydrogenation crosslinking condensation reaction between a diorganopolysiloxane having hydroxyl end groups and a diorganopolysiloxane containing at least one hydrogen bonded to the silicon;
- by crosslinking condensation reaction of a diorganopolysiloxane having hydroxyl end groups and of a hydrolysable organopolysilane;
- by thermal crosslinking of an organopolysiloxane; and
- by crosslinking of an organopolysiloxane by high-energy radiation.

8. Composition according to any one of the preceding claims, **characterized in that** the organopolysiloxane elastomer is obtained by crosslinking addition reaction (A) of a diorganopolysiloxane containing at least two hydrogen atoms each bonded to a different silicon atom, and (B) of a diorganopolysiloxane having at least two ethylenically unsaturated groups bonded to the silicon, especially in the presence (C) of a platinum catalyst.

9. Composition according to any one of the preceding claims, **characterized in that** the organopolysiloxane elastomer is obtained by reaction of a dimethylpolysiloxane having dimethylvinylsiloxy end groups and of a methylhydrogenpolysiloxane having trimethylsiloxy end groups, in the presence of a platinum catalyst.

10. Composition according to any one of the preceding claims, **characterized in that** the organopolysiloxane elastomer is a dimethicone/vinyldimethicone copolymer (or polysilicone-11).

11. Composition according to any one of the preceding claims, **characterized in that** it comprises a crosslinked amphiphilic polymer.

12. Composition according to any one of the preceding claims, **characterized in that** the amount, as active material, of organopolysiloxane elastomer(s) ranges from 0.01 to 10% by weight relative to the total weight of the composition, and preferably from 0.5 to 7% by weight and better still from 1 to 4% by weight relative to the total weight of the composition.

13. Composition according to any one of the preceding claims, **characterized in that** the amount of oily phase is less than 40% of the total weight of the composition, preferably less than or equal to 35% by weight.

14. Composition according to any one of the preceding claims, **characterized in that** the emulsion has an average size of the oily globules ranging from 15 to 500 microns, preferably from 15 to 300 µm and better still from 15 to 250 µm.

15. Composition according to any one of the preceding claims, **characterized in that** it comprises an emulsifier content of less than or equal to 1% by weight relative to the weight of the composition, preferably less than or equal to 0.5% by weight and better still less than or equal to 0.1% by weight.

16. Method for the cosmetic treatment of keratin materials, **characterized in that** a cosmetic composition according to any one of Claims 1 to 15 is applied to the keratin materials.
